(19) Europäisches Patentamt / European Patent Office / Office européen des brevets

(11) **EP 2 482 732 B1**

(12) **EUROPEAN PATENT SPECIFICATION**

(45) Date of publication and mention
of the grant of the patent:
**18.01.2017 Bulletin 2017/03**

(21) Application number: **10771221.8**

(22) Date of filing: **22.09.2010**

(51) Int Cl.:
***A61B 8/13*** (2006.01)

(86) International application number:
**PCT/JP2010/005749**

(87) International publication number:
**WO 2011/039979 (07.04.2011 Gazette 2011/14)**

(54) **BIOLOGICAL INFORMATION PROCESSING APPARATUS AND BIOLOGICAL INFORMATION PROCESSING PROGRAM**

VORRICHTUNG ZUR VERARBEITUNG BIOLOGISCHER INFORMATIONEN UND PROGRAMM ZUR VERARBEITUNG BIOLOGISCHER INFORMATIONEN

APPAREIL DE TRAITEMENT D'INFORMATIONS BIOLOGIQUES ET PROGRAMME DE TRAITEMENT D'INFORMATIONS BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **30.09.2009 JP 2009227241**

(43) Date of publication of application:
**08.08.2012 Bulletin 2012/32**

(73) Proprietor: **Canon Kabushiki Kaisha**
**Tokyo 146-8501 (JP)**

(72) Inventor: **YUKI, Osamu**
**Ohta-ku, Tokyo (JP)**

(74) Representative: **TBK**
**Bavariaring 4-6**
**80336 München (DE)**

(56) References cited:
**WO-A1-2008/081558     US-A1- 2005 288 581**

## Description

## Technical Field

The present invention relates to a biological information processing apparatus and a biological information processing program using a photoacoustic effect or ultrasonic waves.

## Background Art

Biological information processing apparatuses using ultrasonic waves have been developed in order to examine the state inside bio-tissues. One example is an apparatus utilizing a photoacoustic effect. This apparatus irradiates lights onto a bio-tissue, and receives ultrasonic waves (photoacoustic waves) generated by the photoacoustic effect based on the light energy. And the received information is analyzed to obtain the substance, position and size of the light absorber inside a biological body, and this information is used for diagnosis. Since a photoacoustic effect is used, this apparatus is also called a PAT (Photo Acoustic Tomography) apparatus.

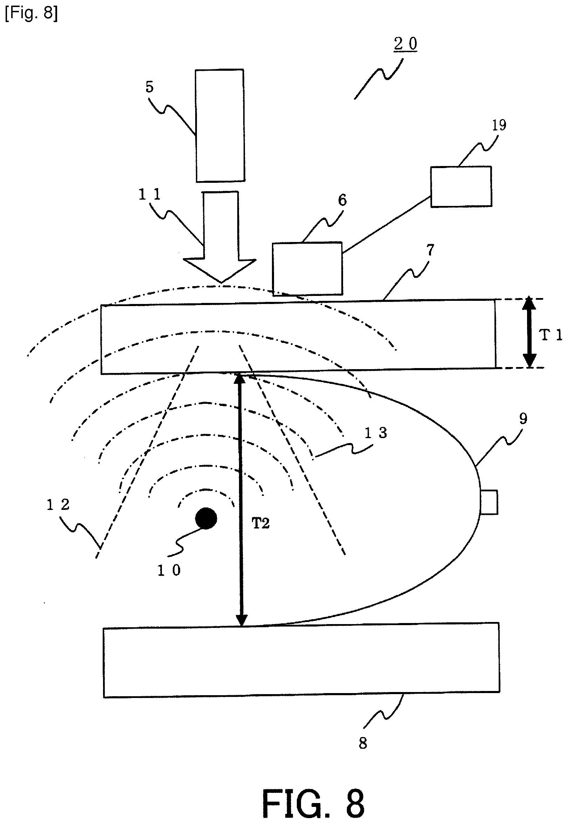

Fig. 8 shows a configuration of a measuring unit 20 of this photoacoustic biological information processing apparatus. The measuring unit 20 has a light source 5, transducer 6, compressing plate 7 and counter compressing plate 8. The transducer 6 is connected with a processing unit 19. The breast 9 of a patient is the subject, and an absorber 10 is inside the subject. The absorber 10 is a cancer, for example, of which characteristic response to irradiated light is different from peripheral bio-tissue. The breast 9 is deformed to be thickness T2 by the compressing plate 7 and the counter compressing plate 8, which are transparent and transmit light. This compressing processing is performed to make a breast 9, which is a bio-tissue and difficult to transmit light, to be thickness T2 which light can reach.

During the measurement, the light source 5 irradiates light 11 onto the breast 9. The light 11 is diffused inside the bio-tissue and becomes diffused light 12. If the diffused light 12 contacts the absorber 10, the absorber 10 expands and contracts, which generates ultrasonic waves 13. The transducer 6 receives the ultrasonic waves 13, converts them into electric signals (analog signals), and sends these signals to the processing unit 19. The processing unit 19 performs such processing as analog-digital conversion, and transfers the data to a CPU. The CPU calculates the phases, determines the position, size and optical characteristics of the absorber 10, and displays a reconstructed image.

Light distribution according to the depth is described using Expression (1).

[Math.1]

$$\phi(d) = \phi0 \times \exp(-\mu eff \times d) \quad \dots \quad (1)$$

where

d :        distance from area, onto which light from the light source is irradiated, to the light absorber in bio-tissue,
$\phi0$ :      light intensity in the irradiated area,
$\mu$eff :    effective attenuation coefficient, and
$\phi$(d) :    light distribution at distance d.

Here, effective attenuation coefficient indicates attenuation characteristic due to light scattering and absorption inside the biological body.

The above mentioned effective attenuation coefficient is described using Expression (2).

[Math.2]

$$\mu eff = [3 \times \mu a \times \{\mu a + \mu s \times (1-g)\}] -1/2 \quad \dots \quad (2)$$

where

$\mu$a :      absorption coefficient,
$\mu$s :      scattering coefficient, and
g :        anisotropy.

These values are different depending on the matter, and in this case values according to the bio-tissue are used.

As Expression (1) and Expression (2) show, the light intensity exponentially attenuates as the distance d in the bio-tissue increases, and therefore it is difficult for the light to reach the deep areas of the bio-tissue.

Another example of the biological information processing apparatus using ultrasonic waves is an apparatus which transmits and receives ultrasonic waves. This ultrasonic biological information processing apparatus transmits the ultrasonic waves, receives the ultrasonic waves reflected from the bio-tissue, and reconstructs the image.

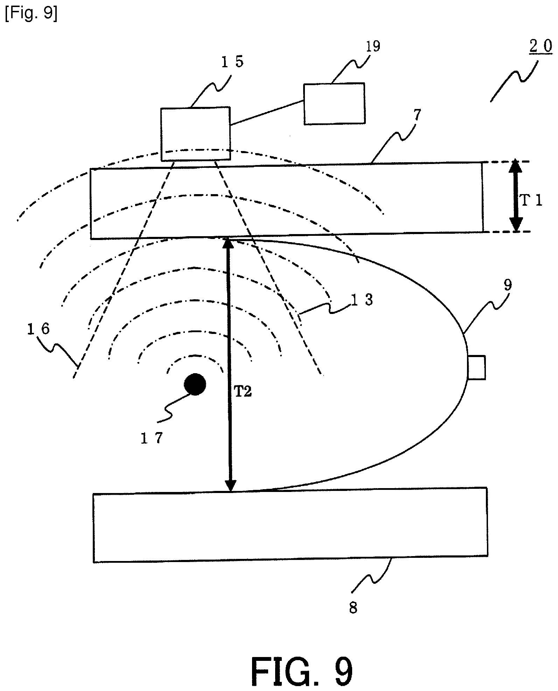

Fig. 9 is a diagram depicting a measuring unit 20 of the ultrasonic biological information processing apparatus. Just like the above mentioned apparatus using a photoacoustic effect, the measuring unit 20 presses the breast 9 by the compressing plate 7 and the counter compressing plate 8, so as to be thickness T2 at which ultrasonic waves can reach. In the breast 9, a measurement target 17, of which characteristic response to ultrasonic waves is different from peripheral tissue, exists. The measuring unit 20 also has a transducer 15. This transducer has a function to transmit ultrasonic waves, in addition to a function to receive ultrasonic waves and convert them into electric signals (analog signals). In other words, the transducer 15 transmits the ultrasonic waves 16 to the breast 9, receives the ultrasonic waves 13 reflected by the measurement target 17 inside the biological body, and sends the electric signals to the processing unit 19. The processing unit 19 performs such processing as analog-digital conversion, and transfers the data to the CPU. The CPU computes data so as to determine the position and size of the measurement target 17, and reconstructs the image.

Document WO 2008/081558 A1 discloses an ultrasound imaging apparatus capable of easily displaying a three-dimensional image included in a region of interest. A display controller causes a display to display a tomographic image, and further causes the display to display a first marker indicating a three-dimensional scan range and a second marker indicating a range to generate three-dimensional image data (a range subjected to rendering) so as to be superimposed on a tomographic image. The second marker is rotatable on the tomographic image in accordance with an instruction by an operator. A transceiver causes an ultrasound probe to scan the three-dimensional scan range specified based on the first marker. An image processor executes rendering on, of data acquired in the scan, data included in the range specified based on the second marker, thereby generating three-dimensional image data. Document US 2005/288581 A1 discloses a combined mammography and ultrasound imaging system. The system includes an ultrasound probe, which transmits ultrasound signals to a breast of a patient and receives reflected ultrasound signals from the breast. The system further includes a first acoustic coupling sheath. A first side of the first acoustic coupling sheath is coupled to a face of the ultrasound probe. The system also includes a mammography compression plate for compressing the breast of the patient. A second acoustic coupling sheath coupled to a side of the mammography compression plate contacts the breast of the patient.

[Summary of Invention]

However in the photoacoustic biological information processing apparatus in Fig. 8, the propagation speed of the irradiated light 11 and the diffused light 12 is negligibly fast, but the speed of the ultrasonic waves 13 is much slower than this. Therefore a number of data obtained during the propagation time required for the ultrasonic waves 13, transmitted from the bio-tissue to pass through the compressing plate 7, cannot be ignored.

A number of obtained data while the ultrasonic waves passing through the compressing plate 7 further increases if a plurality of pieces of data are obtained in parallel using transducers arrayed in a two-dimensional matrix. In the case of transferring the plurality of pieces of obtained data to a CPU, it takes a long time because a number of data that can be transferred in parallel via a transmission path, such as a cable, is limited.

In the ultrasonic biological information processing apparatus in Fig. 9, it takes considerable propagation time when the ultrasonic waves 16 are transmitted from the transducer 15, and when reflected ultrasonic waves 13 are received. Therefore when the transducer 15 transmits the ultrasonic waves 16 and receives the ultrasonic waves 13 reflected from the measurement target 17, a number of obtained data, when ultrasonic waves are passing through the compressing plate 7, cannot be ignored. Further, if transducers arrayed in a two-dimensional matrix are used, a number of obtained data and data transfer time increase, which is the same as the case of the photoacoustic biological information processing apparatus.

A portion, which is not related to the characteristics of the absorber 10 or the measurement target 17 to be determined, such as the compressing plate 7 in the above mentioned period of the ultrasonic waves passing through, is called the ineffective area in the present invention. The operator of the apparatus can determine not only the compressing plate, but also an arbitrary area (e.g. portion in the breast where cancer does not exist) as the ineffective area. The data obtained when the ultrasonic waves are passing through the ineffective area is called the ineffective data. In such a case, as the number of obtained ineffective data increases, the storage capacity of unnecessary data and the data transfer volume to the CPU increase, and the signal waveform start position searching time increases when the image is reconstructed in the CPU. These problems occur even for a single bit transducer, but occur more conspicuously if the transducers are arrayed in a two-dimensional matrix.

With the foregoing in view, it is an object of the present invention to provide a biological information processing apparatus and a biological information processing program which enable efficient data recording and data transfer.

According to an aspect of the present invention, there is provided a biological information processing apparatus as defined in claim 1.

According to another aspect of the present invention, there is provided a biological information processing program as defined in claim 7.

According to the biological information processing apparatus and the biological information processing program of the present invention, efficient data recording and data transfer are enabled.

Further features of the present invention will become apparent from the following description of exemplary embodiments with reference to the attached drawings.

**Brief Description of Drawings**

[fig.1]Fig. 1 is a diagram depicting a processing unit of a biological information processing apparatus of Embodiment 1.

[fig.2]Fig. 2 is a diagram depicting a control timing of the biological information processing apparatus of Embodiment 1.

[fig.3]Fig. 3 is a diagram depicting a processing unit of a biological information processing apparatus of Embodiments 2 and 3.

[fig.4]Fig. 4 is a diagram depicting a control timing of the biological information processing apparatus of Embodiment 2.

[fig.5]Fig. 5 is a diagram depicting a control timing of a biological information processing apparatus of Embodiment 3.

[fig.6]Fig. 6 is a diagram depicting a configuration of a photoacoustic biological information processing apparatus of Embodiment 2.

[fig.7]Fig. 7 is a diagram depicting a configuration of a ultrasonic biological information processing apparatus of Embodiment 3.

[fig.8]Fig. 8 is a diagram depicting a measuring unit of the photoacoustic biological information processing apparatus of prior art and Embodiment 1.

[fig.9]Fig. 9 is a diagram depicting a configuration of an ultrasonic biological information processing apparatus of prior art.

[fig.10]Fig. 10 is a flow chart depicting data acquisition of the biological information processing apparatus of Embodiment 2.

**Description of Embodiments**

(Embodiment 1)

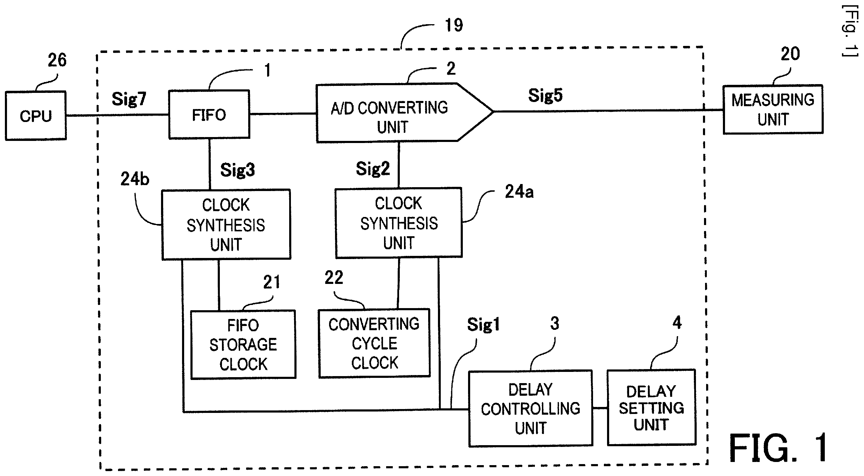

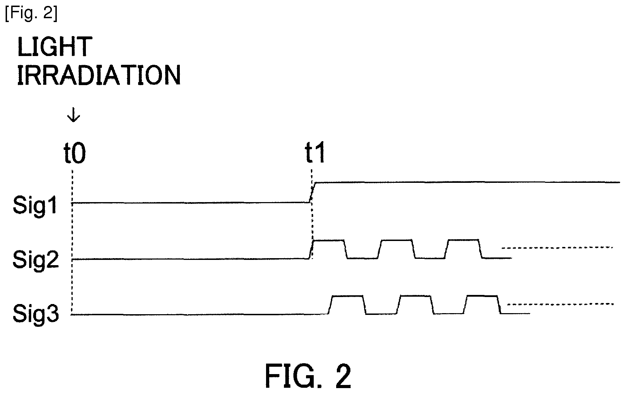

Embodiment 1 of the biological information processing apparatus according to the present invention will now be described in detail, with reference to Fig. 1, Fig. 2 and the above mentioned Fig. 8.

Fig. 1 shows a configuration of a processing unit 19 of the biological information processing apparatus used for the present embodiment. The processing unit 19 is connected to a signal wiring Sig 5 from a measuring unit 20 shown in Fig. 8, and receives a signal from a transducer 6. The processing unit 19 comprises a first-in-first-out storage apparatus 1 (hereafter FIFO), analog-digital converting unit 2, delay controlling unit 3, and delay setting unit 4. The processing unit 19 further comprises an FIFO storage clock 21, which can output a control signal, and a conversion cycle clock 22, which outputs a control signal. A clock synthesis unit 24a is connected to the FIFO 1, and a clock synthesis unit 24b is connected o the analog-digital converting unit 2. The processing unit 19 is connected to a CPU 26 via a signal wiring Sig 7. The CPU 26 is an information processing apparatus to perform computing processing and display processing, and is constituted by a computer or the like.

The transducer 6 corresponds to the conversion element of the present invention. The FIFO 1 corresponds to the first memory of the present invention. The delay setting unit 4 corresponds to the second memory of the present invention. The delay controlling unit 3 corresponds to the timer of the present invention. The CPU 26 corresponds to the signal processing unit of the present invention.

The measuring unit 20 has a configuration similar to that described above with reference to Fig. 8. In the present embodiment, it is assumed that the transducer 6 is constituted by 352 pixels (elements) arrayed in a two-dimensional matrix. The transducer 6 can receive ultrasonic waves (photoacoustic waves) generated by the photoacoustic effect of the irradiated light from the light source. The concept of the ineffective area and the ineffective data is the same as those mentioned above. In other words, an area which the operated determined that recording thereof is unnecessary (e.g. normal bio-tissue free from cancer) and an area which obviously need not be recorded, such as a compressing plate, out of the path of ultrasonic waves, is called the ineffective area. A signal obtained during a period, when the ultrasonic waves transmitted from a point in the ineffective area can be received by the transducer 6 (or digitally converted data of this signal), is called the ineffective data.

The compressing plate 7 corresponds to the plate-like member, and plays a role of securing a breast 9, which is the subject (bio-tissue). The compressing plate 7 in the present embodiment has an optical characteristic to transmit through the light irradiated from the light source 5 so that the light reaches the breast (typically transparent).

When the apparatus is activated and light is irradiated, the transducer 6 receives the ultrasonic waves, and converts them into analog signals. The analog-digital converting unit 2 performs analog-digital conversion for the analog signals received via Sig 5. If ultrasonic waves are transmitted from the entire depth (that is, T1 and T2), as in the case of prior art, sampling (ultrasonic wave reception and analog-digital conversion) is repeated immediately after the light is irradiated until the ultrasonic waves, generated at the deepest portion of the breast, reach the transducer 6. If a number of times of sampling per one measurement (light irradiation) is 1000 times, the acquired data volume is as follows: the data volume to be recorded in the FIFO 1 per one measurement is 16 (bit) * 1000 (number of data) * 352 (number of pixels) = 5632000 bits, where the digital data length of one data is 16 bits.

This data is transferred to the CPU 26 via the FIFO 1. In the present embodiment, the transfer speed is assumed to be 100 Mbps, which is the LAN standard. Then it takes about 56.3 milliseconds for one measurement. If the propagation speed of the sound wave in the compressing plate 7 is 2200 m/s, and the thickness T1 is 1 cm in the present embodiment, then it takes about 4.5 microseconds for the ultrasonic wave 13 to pass through the compressing plate 7.

In the present embodiment, the analog-digital conversation rate is assumed to be 20 MHz. Therefore a number of analog-digital converted ineffective data, which corresponds to the period of the ultrasonic wave 13 passing through the compressing plate 7, is 4.5 microseconds * 20 MHz, that is 90.

According to a conventional biological information processing apparatus, the ineffective data, while the ultrasonic waves generated from the surface layer of the bio-tissue closest to the transducer passes through the compressing plate, is also recorded in the FIFO 1. This ineffective data is transferred together when data is transferred from the processing unit to the CPU. Therefore the high-speed data transfer standard must be selected to guarantee the transfer volume, which increases cost. This data transfer time from the FIFO to the CPU is about 5 milliseconds at 100 Mbps, which is the LAN standard, and generates a processing delay. The signal waveform start position searching time is also an extra requirement when the CPU reconstructs the image.

Therefore in the present embodiment, the analog-digital conversion unit controls so that data, during the period when the ultrasonic waves are passing through the ineffective area (the compressing plate 7 in this embodiment), is not analog-digital converted. This will be described with reference to the timing chart in Fig. 2. In Fig. 2, t0 is a timing of starting irradiating light, and t1 is a timing of starting collecting data, which is determined corresponding to the sound wave propagation period in the ineffective area, that is 4.5 microseconds.

First, 4.5 milliseconds, that is the time for the ultrasonic waves 13 to pass through the compressing plate 7, determined based on the sound speed and the thickness of the compressing plate 7, is stored in the delay setting unit 4 in advance. The delay controlling unit 3 counts this stored time, and generates a control signal Sig 1, which is t1 delayed from the light irradiation start time t0.

The clock synthesis unit 24a combines the time generated by the conversion cycle clock 22 and the above mentioned Sig 1, and supplies it to the analog-digital converting unit 2 as Sig 2. The analog-digital converting unit 2 starts analog-digital conversion at the rise of Sig 2. Thus the activation of the analog-digital converting unit 2 can be controlled.

The clock synthesis unit 24b combines the timing generated by the FIFO storage clock 21 and the above mentioned Sig 1, and supplies the result to the FIFO as Sig 3. The FIFO 1 starts recording data supplied from the analog-digital converting unit 2 at the rise of Sig 3.

Since the processing unit of the biological information processing apparatus of the present embodiment has this configuration, execution of the analog-digital conversion is stopped for the data which the transducer 6 obtained when the ultrasonic waves passed through the ineffective area. As a result, unnecessary data is not recorded in the memory, and the memory resource can be effectively used. Unnecessary data is not transferred to the CPU either, so cost of the communication apparatus, data transfer time and data processing time can be decreased.

(Embodiment 2)

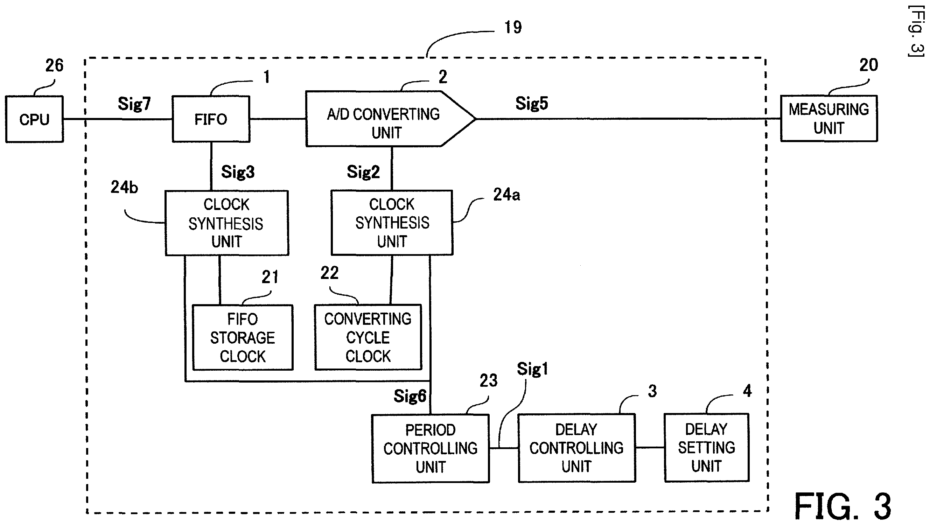

Embodiment 2 of the biological information processing apparatus according to the present invention will now be described in detail, with reference to Fig. 3, Fig. 4 and Fig. 6. The apparatus of this embodiment is a photoacoustic biological information processing apparatus, just like the case of Embodiment 1.

Fig. 3 shows a configuration of a processing unit 19 of the biological information processing apparatus used for the present embodiment. A composing element having the same function as Fig. 1 is denoted with a same number, for which description is omitted. The processing unit 19 of the present embodiment has a period controlling unit 23 which outputs a control signal on analog-digital conversion and recording to the FIFO.

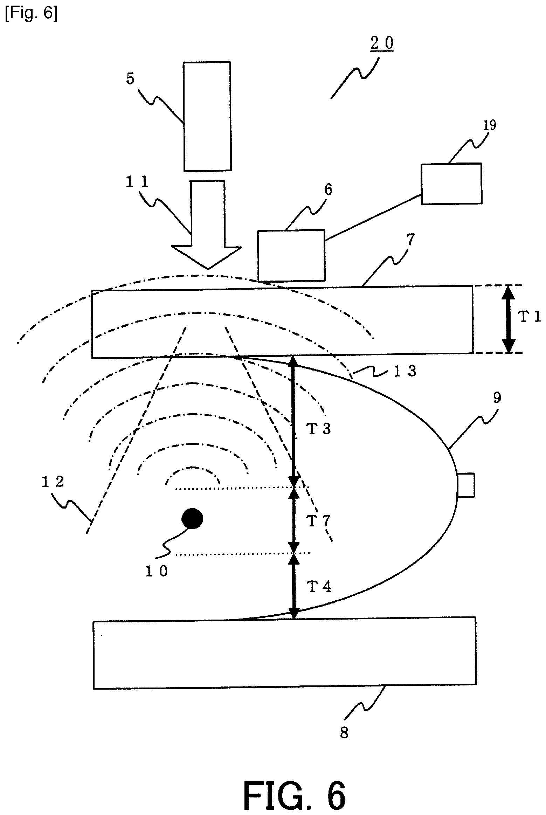

Fig. 6 shows a configuration of the measuring unit 20 used for the present embodiment. In the present embodiment, the arrangement and function of each block are the same as Fig. 8 (Embodiment 1), but the concept on the ineffective area is different. In Fig. 8, the thickness T2 of the breast 9 is divided into three, T3, T4 and T7. The thickness T7 is a portion including an absorber 10, which is the measurement target area. In this case, T3 and T4 are regarded as the ineffective areas for inspection, although they are inside the biological body.

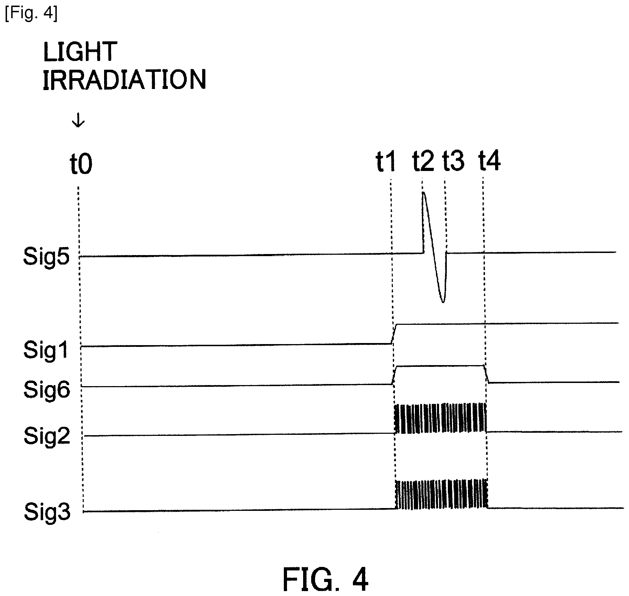

A method of handling data on the ineffective area according to the present embodiment will be described with reference to the timing chart in Fig. 4. First, in the period controlling unit 23, which is the characteristic of the present embodiment, a number of times of analog-digital conversion and a number of times of FIFO storing are set. In the present embodiment, a number of times of the analog-digital converting unit 2 converting the analog signals into digital signals is set to 200 for one measurement (light irradiation), and a number of times of storing data to the FIFO storage apparatus 1 is also set to 200.

Then the delay setting unit 4 sets time for the delay controlling unit 3 based on the timing t0 when the light irradiation is started in Fig. 4. At this time, in order to specify the depth T7 where the light absorber 10 exists, the depth is measured while adjusting the delay time of the delay controlling unit 3 using the setting by the delay setting unit 4. In the case of a multi-modality diagnosis with X-ray and ultrasonic diagnosis, the general location of the absorber could be specified in advance. By specifying the measurement target area like this, a number of times of the analog-digital conversion unit 2 converting from analog signals into digital signals, and a number of times of storing the digital data to the FIFO storage apparatus 1, can be decreased.

The delay setting unit 4 inputs 24.5 microseconds to the delay controlling unit 3, which is the sum of about 4.5 microseconds, which is time required for the ultrasonic waves 13 to pass through the thickness T1 of the compressing plate 7, and 20 microseconds, which is time required for the ultrasonic waves 13 to pass through the area T3, which is ineffective for inspection. Then the delay controlling unit 3 counts this time as shown in Fig. 4, and generates a control signal Sig 1, in which the start time of analog-digital conversion is delayed t1 from the light irradiation start time t0. During the period from t1 to t4, which is equivalent to T7, Sig 5, including the peak corresponding to the ultrasonic waves generated from the absorber 10, is detected.

In the period controlling unit 23, a number of times of analog-digital conversion and a number of times of storing data to the FIFO, during the period from t1 to t4, that are both 200, are set. Here the period is set in Sig 1, in which the light irradiation timing t0 is delayed, and the control signal Sig 6 is output.

Then the clock synthesis unit 24a combines the timing generated by the conversion cycle clock 22 and the above mentioned Sig 6, and supplies the combined signal to the analog-digital converting unit 6 as Sig 2. The analog-digital converting unit 2 starts analog-digital conversion at the rise the Sig 2, and converts the analog signal to the digital signal.

The clock synthesis unit 24b combines the timing generated by the FIFO storage clock 21 and the above mentioned Sig 6, and supplies the combined signal to the FIFO 1 as Sig. 3. The FIFO 1 starts recording data, supplied from the analog-digital converting unit, at the rise of Sig 3.

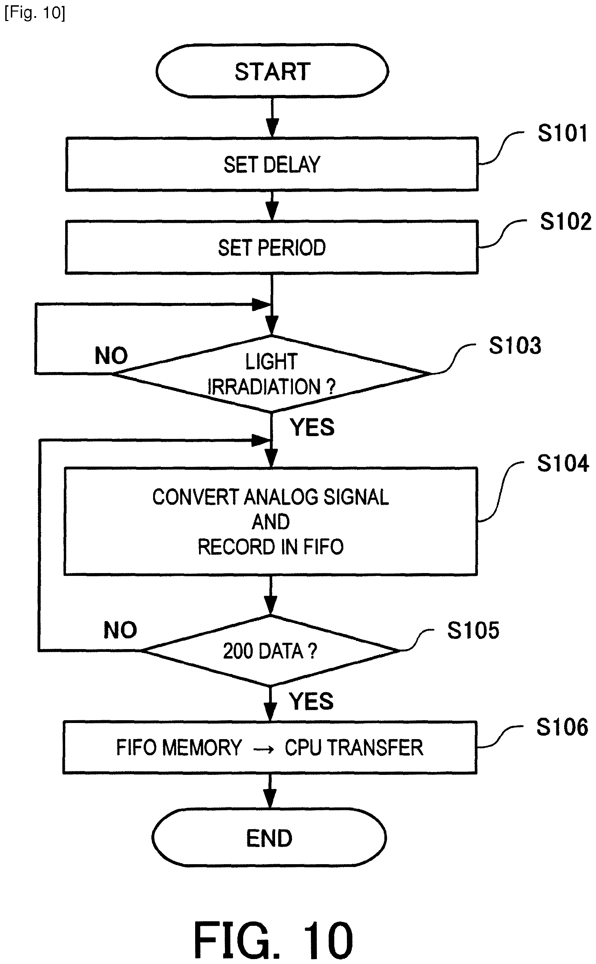

Fig. 10 shows the data acquisition flow in this one cycle of measurement. After processing starts, the delay time is set in the delay setting unit 4 in step S101. Then in step S102, the period related to the analog-digital conversion is set in the period controlling unit 23. Then in step S103, it is determined whether light was irradiated or not. If S103 = NO, the determination is repeated after standing by for a predetermined time. If S103 = YES, processing advances to step S104, and the analog signal, which the transducer 6 converted from an ultrasonic wave, is analog-digital converted and stored in FIFO 1. At this time, the control signal is generated based on the above mentioned delay time and information being set in the period controlling unit, so unnecessary data is not analog-digital converted or stored in the FIFO. Then in step S105, it is determined whether 200 data, which is a number of data stored in the period controlling unit 23, is processed or not. If S105 = NO, processing returns to S104, and the next data is acquired. If S105 = YES, the data recorded in the FIFO 1 is transferred to the CPU 6. Thus the present invention can be implemented as the biological information processing program which controls the processing apparatus and measuring apparatus so as to execute each processing.

In the present embodiment, the acquired data volume per one measurement is 16 (bit) * 200 (number of data) * 352 (number of pixels) = 1126400 bits. To transfer this data from the FIFO 1 to the CPU 26, it takes about 11.3 milliseconds if 100 Mbps, which is the LAN standard, is used. On the other hand, if the data on the entire depth, that is the total, of the thickness T2 of the breast 9 and the thickness T1 of the compressing plate 7, shown in Fig. 8, is acquired, the transfer data volume is 16 (bit) * 1000 (number of data) * 352 (number of pixels) = 5632000 bits. To transfer this data from the FIFO 1 to the CPU 26, it takes about 56.3 milliseconds if 100 Mbps, which is the LAN standard, is used, just like the above case. In other words, according to the means of the present embodiment, the data transfer time can be 1/5. By decreasing the analog-digital conversion target data, the capacity of the FIFO 1 can also be decreased.

In the case of reconstructing the image for diagnosis on the display in this biological information processing apparatus, the data can be transferred in 11.3 milliseconds per one measurement if the means of the present embodiment is used. This means that a display at 60 Hz (16.7 milliseconds), which is the standard display update speed, is easily implemented. If the data is acquired for the entire depth, that is, the total of the thicknesses T3 and T4 of the subject and the thickness T1 of the compressing plate 7, as shown in Fig. 6, a display at the standard display update speed cannot be executed.

(Embodiment 3)

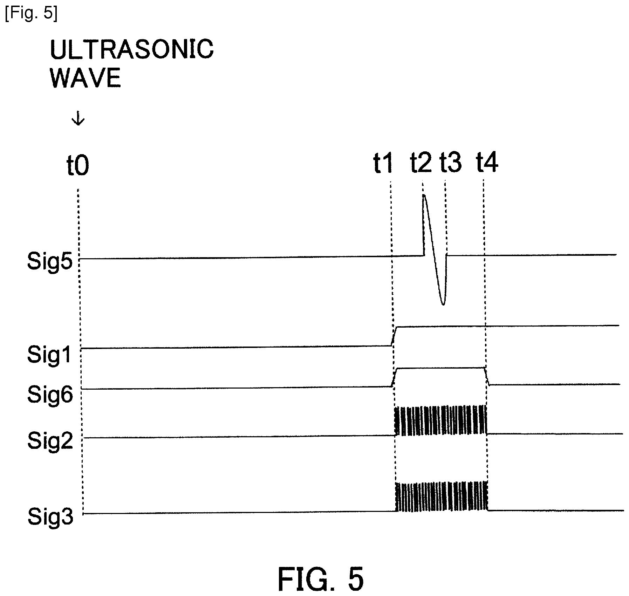

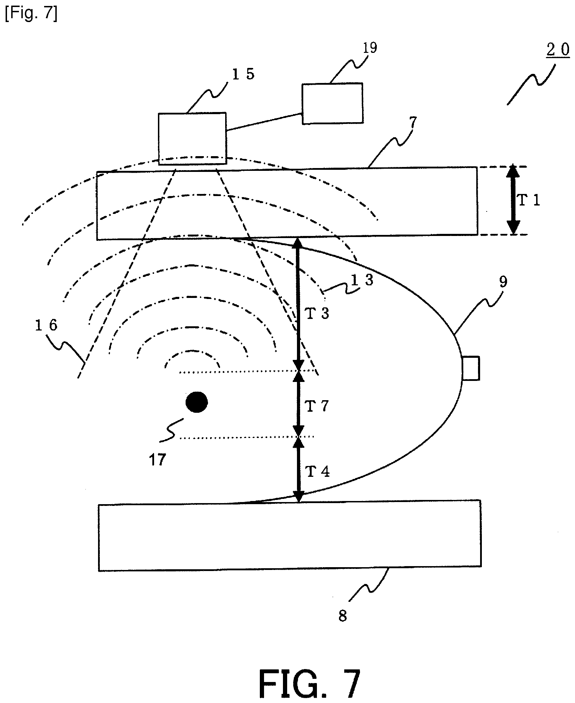

Embodiment 3 of the biological information processing apparatus according to the present invention will now be described with reference to Fig. 5, Fig. 7 and the above mentioned Fig. 3. The apparatus of the present embodiment transmits ultrasonic waves and receives the ultrasonic waves reflected from the bio-tissue. In the above mentioned Embodiments 1 and 2, the speed of light irradiated onto the bio-tissue was handled as being negligibly fast. In the present embodiment, however, the speed of the ultrasonic waves during transmission is also considered.

A measuring unit 20 in the present embodiment shown in Fig. 7 has a configuration similar to a conventional apparatus using ultrasonic waves, which was described with reference to Fig. 9. However the thickness of the breast 9, which is a subject, is considered separately for the range T7, which includes the measurement target 17, and T3 and T4, which are ineffective areas for inspection, although they are inside the biological body. Information on the period when the ultrasonic waves pass through the compressing plate 7 is also processed as an ineffective area. A transducer 15 has a function to transmit the ultrasonic waves and receives the reflected ultrasonic waves.

A processing unit 19 of the present embodiment has a similar configuration as that described with reference to Fig. 3. An analog-digital converting unit 2 of the present embodiment receives a signal Sig 5 from the transducer 15 of the measuring unit.

The processing according to the present embodiment will be described with reference to the timing chart shown in Fig. 5. The delay setting unit 4 sets the time for the delay controlling unit 3 based on the timing t0, when the ultrasonic waves are irradiated, as a reference. In the period controlling unit 23, a number of times of analog-digital conversion and a number of times of storing data to the FIFO are set. In the present embodiment, the number of times of the analog-digital converting unit 2 executing the analog-digital conversion is 200, and the number of times of the FIFO 1 storing data is also 200. At this time, in order to specify the depth T7, where the light absorber 10 exists, the depth is measured while adjusting the delay time of the delay controlling unit 3 using the delay setting unit 4. In the case of a multi-modality diagnosis with X-ray and ultrasonic diagnosis, a general setting value could be specified in advance. By specifying the measurement target area like this, a number of times of the analog-digital conversion and a number of times of storing the data to the FIFO 1 can be decreased.

In the present embodiment, the delay setting unit 4 sets about 4.5 microseconds * 2 times in the delay controlling unit 3, as a time required for the ultrasonic waves 16 transmitted from the transducer 15 propagating the thickness T1 of the compressing plate 7, and returning. The delay setting unit 4 also sets 20 microseconds * 2 times in the delay controlling unit 3, as a time required for the ultrasonic waves propagating the ineffective area T3 for inspection, and returning. In other words, a total of 49 microseconds is set. Then the delay controlling unit 3 counts the setting time, as shown in Fig. 5, and generates a control signal Sig 1 in which the start time of analog-digital conversion is delayed t1 from the light irradiation start time t0. During the period from t1 to t4, which is equivalent to T7, Sig 5, including the peak corresponding to the ultrasonic waves generated from the measurement target 10, is detected.

In the period controlling unit 23, a number of timings of analog-digital conversion, and a number of times of storing data in the FIFO, during the period from t1 to t4, that are both 200, are set. Then the period is set to Sig 1 in which the light irradiation timing t0 is delayed, and the control signal Sig 6 is output. Then the clock synthesis unit 24a combines the timing generated by the conversion cycle clock 22 and the above mentioned Sig 6, and supplies the combined signal to the analog-digital converting unit 2 as Sig 2. The analog-digital converting unit 2 starts analog-digital conversion at the rise of Sig 2. The clock synthesis unit 24b combines the timing generated by the FIFO storage clock 21 and the above mentioned Sig 6, and supplies the combined signal to the FIFO 1 as Sig 3. The FIFO 1 receives and stores data from the analog-digital converting unit 2 at the rise of Sig 3. The data acquisition flow at this time is the same as that described above with reference to Fig. 10.

In the present embodiment, the acquired data volume per one measurement is 16 (bit) * 200 (number of data) * 352 (number of pixels) = 1126400 bits. To transfer this data from the FIFO 1 to the CPU 26, it takes about 11.3 milliseconds if 100 Mbps, which is the LAN standard, is used.

On the other hand, if the data on the entire depth, that is the total of the thickness T2 of the subject and the thickness T1 of the compressing plate 7, shown in Fig. 9, is acquired, the data volume per one measurement is 16 (bit) * 1000 (number of data) * 352 (number of pixels) = 5632000 bits. To transfer this data from the FIFO 1 to the CPU 26, it takes about 56.3 milliseconds if 100 Mbps, which is the LAN standard, is used. Therefore the data transfer speed improves even more if the thicknesses T3 and T4 of the breast 9, which is the subject, and the thickness T1 of the compressing plate 7, are set as the ineffective areas, as shown in Fig. 7. In the case of the CPU reconstructing the image for diagnosis by video, this data transfer speed of the present embodiment can easily enable a display at 60 Hz (16.7 milliseconds), which is the standard display update speed.

(Embodiment 4)

In Embodiments 1 to 3 mentioned above, data is reduced by controlling the timing of the data on the ineffective area so that analog-digital conversion is not performed. In the present embodiment, a method for performing analog-digital conversion for the data on the ineffective area, but not storing the result in the FIFO, will be described. This control can be implemented by changing the operation of the delay controlling unit 3 and the period controlling unit 23 of the processing unit 19.

For example, in the processing unit 19, which was described with reference to Fig. 1 in Embodiment 1, the delay controlling unit 3 transmits the delay time in the ineffective area T1 only to the clock synthesis unit 24b, which is connected to the FIFO storage clock 21. The clock synthesis unit 24b combines the timing from the FIFO storage lock 21 and Sig 1 from the delay controlling unit, and supplies the combined signal to the FIFO 1. On the other hand, the analog-digital converting unit 2 digitizes the signal received from the transducer and continues sending it to the FIFO 1. The FIFO 1 stores data while eliminating the data on the ineffective area, according to Sig 3 from the clock synthesis unit 24b.

By this configuration as well, the amount of memory resources required by the FIFO 1 and data transfer volume to the CPU 26 can be decreased. In Embodiments 2 and 3 described with reference to Fig. 3 as well, the effect of decreasing the memory resources to be used and data transfer volume can be implemented by the FIFO side controlling the timing to store the data in the same manner.

The processing unit 19 described in the above embodiments is not limited to the above description, but can take various configurations. For example, as the information on the ineffective area that is set in the delay setting unit, separate data may be provided so that the specification on the range of the ineffective area, by input from the operator, is accepted, and the propagation time of the ultrasonic waves corresponding to this distance is calculated. The memory for storing the analog-digital converted data is not limited to the FIFO memory, but may be another memory apparatus. The connection of the processing unit 19 and the CPU 26 is not limited to the LAN standard, but other means, including radio communication and various cables, may be used according to the configuration of the computer apparatus including the CPU.

(Other Embodiments)

Aspects of the present invention can also be realized by a computer of a system or apparatus (or devices such as a CPU or MPU) that reads out and executes a program recorded on a memory device to perform the functions of the above-described embodiment(s), and by a method, the steps of which are performed by a computer of a system or apparatus by, for example, reading out and executing a program recorded on a memory device to perform the functions of the above-described embodiment(s). For this purpose, the program is provided to the computer for example via a network or from a recording medium of various types serving as the memory device (e.g., computer-readable medium).

**Claims**

1. A biological information processing apparatus, comprising:

    a conversion element (6) adapted to receive an ultrasonic wave transmitted from a subject and convert the ultrasonic wave into an analog signal;
    an analog-digital converting unit (2) adapted to convert the analog signal into a digital signal;
    a first memory (1) adapted to record the digital signal;
    a signal processing unit (26) adapted to reconstruct an image using the digital signal recorded in the first memory (1);
    a second memory (4) adapted to record information on an ineffective area, which is an area for which it is determined that information need not be recorded, out of areas through which the ultrasonic waves pass; and
    a controlling unit (3) adapted to control activation of the analog-digital converting unit (2) or recording to the first memory (1),

    **characterized in that**
    the controlling unit (3) stops the activation of the analog-digital converting unit (2) or recording to the first memory (1) in a period in which the analog signal or the digital signal based on an ultrasonic wave from the ineffective area can be received based on the information recorded in the second memory (4).

2. The biological information processing apparatus according to Claim 1, further comprising a plate-like member (7) adapted to secure the subject and disposed between the subject and the conversion element (6), wherein the ineffective area is an area where the plate-like member (7) exists.

3. The biological information processing apparatus according to Claim 1, wherein

the ineffective area is an area inside the subject excluding a measurement target area determined by an operator.

4. The biological information processing apparatus according to Claim 1, wherein
the controlling unit (3) is a timer which generates a timing to control the activation of the analog-digital converting unit (2) based on the information recorded in the second memory (4).

5. The biological information processing apparatus according to any one of Claim 1 to 4, further comprising a light source (5) adapted to irradiate light onto the subject, wherein
the ultrasonic wave is a photoacoustic wave which is generated from the subject by the light.

6. The biological information processing apparatus according to any one of Claims 1 to 4, further comprising an element (15) adapted to transmit an ultrasonic wave to the subject, wherein
the ultrasonic wave received by the conversion element is a reflected wave of the transmitted ultrasonic wave.

7. A biological information processing program for causing a processing unit to execute:

a step of receiving an analog signal which is generated by a conversion element converting an ultrasonic wave transmitted from a subject;
an analog-digital converting step of converting the analog signal into a digital signal;
a recording step of recording the digital signal into a first memory;
a step of reconstructing an image using the digital signal recorded in the first memory;
a step of recording, to a second memory, information on an ineffective area, which is an area for which it is determined that information need not be recorded, out of areas through which the ultrasonic wave passes; and
a controlling step of controlling execution of the analog-digital converting step or execution of the recording step,

**characterized in that**
the controlling step stops the execution of the analog-digital converting step or execution of the recording step in a period in which the analog signal or the digital signal based on an ultrasonic wave from the ineffective area can be received based on the information recorded in the second memory.

**Patentansprüche**

1. Vorrichtung zur Verarbeitung biologischer Informationen, mit:

einem Umwandlungselement (6), das dazu angepasst ist, eine Ultraschallwelle, die von einem Subjekt übertragen wird, zu empfangen, und die Ultraschallwelle in ein analoges Signal umzuwandeln;
einer Analog-Digital-Umwandlungseinheit (2), die dazu angepasst ist, das analoge Signal in ein digitales Signal umzuwandeln;
einem ersten Speicher (1), der dazu angepasst ist, das digitale Signal aufzuzeichnen;
einer Signalverarbeitungseinheit (26), die dazu angepasst ist, ein Bild unter Verwendung des digitalen Signals, das in dem ersten Speicher (1) aufgezeichnet ist, zu rekonstruieren;
einem zweiten Speicher (4), der dazu angepasst ist, Informationen über einen ineffektiven Bereich aufzuzeichnen, der ein Bereich ist, für den bestimmt ist, dass keine Informationen aufgezeichnet werden müssen, von den Bereichen, die die Ultraschallwellen durchlaufen; und
einer Steuerungseinheit (3), die dazu angepasst ist, eine Aktivierung der Analog-Digital-Umwandlungseinheit (2) oder ein Aufzeichnen auf dem ersten Speicher (1) zu steuern,

**dadurch gekennzeichnet, dass**
die Steuerungseinheit (3) die Aktivierung der Analog-Digital-Umwandlungseinheit (2) oder ein Aufzeichnen auf dem ersten Speicher (1) in einer Periode, in der das analoge Signal oder das digitale Signal basierend auf einer Ultraschallwelle von dem ineffektiven Bereich empfangen werden kann, stoppt, basierend auf den Informationen, die in dem zweiten Bereich (4) aufgezeichnet sind.

2. Vorrichtung zur Verarbeitung biologischer Informationen gemäß Anspruch 1, weiterhin mit einem plattenähnlichen Element (7), das dazu angepasst ist, das Subjekt zu sichern, und das zwischen dem Subjekt und dem Um-wandlungselement (6) angeordnet ist, wobei

der ineffektive Bereich ein Bereich ist, in dem das plattenähnliche Element (7) vorhanden ist.

3. Vorrichtung zur Verarbeitung biologischer Informationen gemäß Anspruch 1, wobei

der ineffektive Bereich ein Bereich innerhalb des Subjekts außer einem Messzielbereich ist, der durch einen Operator bestimmt wird.

4. Vorrichtung zur Verarbeitung biologischer Informationen gemäß Anspruch 1, wobei

die Steuerungseinheit (3) ein Zeitnehmer ist, der eine Zeitsteuerung erzeugt, um die Aktivierung der Analog-Digital-Umwandlungseinheit (2) basierend auf den Informationen, die in dem zweiten Speicher (4) aufgezeichnet sind, zu steuern.

5. Vorrichtung zur Verarbeitung biologischer Informationen gemäß einem der Ansprüche 1 bis 4, weiterhin mit einer Lichtquelle (5), die dazu angepasst ist, Licht auf das Subjekt auszustrahlen, wobei

die Ultraschallwelle eine fotoakustische Welle ist, die von dem Subjekt durch das Licht erzeugt wird.

6. Vorrichtung zur Verarbeitung biologischer Informationen gemäß einem der Ansprüche 1 bis 4, weiterhin mit einem Element (15), das dazu angepasst ist, eine Ultraschallwelle an das Subjekt zu übertragen, wobei

die Ultraschallwelle, die durch das Umwandlungselement empfangen wird, eine reflektierte Welle der übertragenen Ultraschallwelle ist.

7. Programm zur Verarbeitung biologischer Informationen, um eine Verarbeitungseinheit zu veranlassen, auszuführen:

einen Schritt des Empfangens eines analogen Signals, das durch ein Umwandlungselement erzeugt wird, das eine Ultraschallwelle, die von einem Subjekt übertragen wird, umwandelt;
einen Analog-Digital-Umwandlungsschritt des Umwandelns des analogen Signals in ein digitales Signal;
einen Aufzeichnungsschritt des Aufzeichnens des digitalen Signals in einem ersten Speicher;
einen Schritt des Rekonstruierens eines Bildes unter Verwendung des digitalen Signals, das in dem ersten Speicher aufgezeichnet ist;
einen Schritt des Aufzeichnens, in einem zweiten Speicher, von Informationen über einen ineffektiven Bereich, welcher ein Bereich ist, für den bestimmt ist, dass keine Informationen aufgezeichnet werden müssen, aus den Bereichen, die die Ultraschallwellen durchlaufen; und
einen Steuerungsschritt des Steuerns einer Ausführung des AnalogDigital-Umwandlungsschritts oder einer Ausführung des Aufzeichnungsschnitts,

**dadurch gekennzeichnet, dass**
der Steuerungsschritt die Ausführung des Analog-DigitalUmwandlungsschritts oder eine Ausführung des Aufzeichnungsschritts in einer Periode, in der das analoge Signal oder das digitale Signal basierend auf einer Ultraschallwelle von dem ineffektiven Bereich empfangen werden kann, stoppt, basierend auf den Informationen, die in dem zweiten Speicher aufgezeichnet sind.

**Revendications**

1. Appareil de traitement d'informations biologiques, comprenant :

un élément de conversion (6) conçu pour recevoir une onde ultrasonique émise à partir d'un sujet et convertir l'onde ultrasonique en un signal analogique ;
une unité de conversion analogique-numérique (2) conçue pour convertir le signal analogique en un signal numérique ;
une première mémoire (1) conçue pour enregistrer le signal numérique ;
une unité de traitement de signal (26) conçue pour reconstruire une image en utilisant le signal numérique enregistré dans la première mémoire (1) ;
une seconde mémoire (4) conçue pour enregistrer des informations sur une zone ineffective, qui est une zone pour laquelle il est déterminé que des informations n'ont pas besoin d'être enregistrées, hors zones à travers lesquelles passent les ondes ultrasoniques ; et
une unité de commande (3) conçue pour commander l'activation de l'unité de conversion analogique-numérique (2) ou l'enregistrement vers la première mémoire (1),

**caractérisé en ce que** :

l'unité de commande (3) arrête l'activation de l'unité de conversion analogique-numérique (2) ou l'enregistrement vers la première mémoire (1) dans une période dans laquelle le signal analogique ou le signal numérique basé sur une onde ultrasonique en provenance de la zone ineffective peut être reçu sur la base des informations enregistrées dans la seconde mémoire (4).

2. Appareil de traitement d'informations biologiques selon la revendication 1, comprenant en outre un élément du type plaque (7) conçu pour fixer le sujet et disposé entre le sujet et l'élément de conversion (6), dans lequel :

la zone ineffective est une zone où l'élément du type plaque (7) existe.

3. Appareil de traitement d'informations biologiques selon la revendication 1, dans lequel :

la zone ineffective est une zone à l'intérieur du sujet à l'exclusion d'une zone cible de mesure déterminée par un opérateur.

4. Appareil de traitement d'informations biologiques selon la revendication 1, dans lequel :

l'unité de commande (3) est un temporisateur qui génère un positionnement temporel pour commander l'activation de l'unité de conversion analogique-numérique (2) sur la base des informations enregistrées dans la seconde mémoire (4).

5. Appareil de traitement d'informations biologiques selon l'une quelconque des revendications 1 à 4, comprenant en outre une source de lumière (5) conçue pour irradier de la lumière sur le sujet, dans lequel :

l'onde ultrasonique est une onde photoacoustique qui est générée à partir du sujet par la lumière.

6. Appareil de traitement d'informations biologiques selon l'une quelconque des revendications 1 à 4, comprenant en outre un élément (15) conçu pour émettre une onde ultrasonique vers le sujet, dans lequel :

l'onde ultrasonique reçue par l'élément de conversion est une onde réfléchie de l'onde ultrasonique émise.

7. Programme de traitement d'informations biologiques pour amener une unité de traitement à exécuter :

une étape consistant à recevoir un signal analogique qui est généré par un élément de conversion convertissant une onde ultrasonique émise à partir d'un sujet ;
une étape de conversion analogique-numérique consistant à convertir le signal analogique en un signal numérique ;
une étape d'enregistrement consistant à enregistrer le signal numérique dans une première mémoire ;
une étape consistant à reconstruire une image en utilisant le signal numérique enregistré dans la première mémoire ;
une étape consistant à enregistrer, vers une seconde mémoire, des informations sur une zone ineffective, qui est une zone pour laquelle il est déterminé que des informations n'ont pas besoin d'être enregistrées, hors zones à travers lesquelles passe l'onde ultrasonique ; et
une étape de commande consistant à commander l'exécution de l'étape de conversion analogique-numérique ou l'exécution de l'étape d'enregistrement,

**caractérisé en ce que** :

l'étape de commande arrête l'exécution de l'étape de conversion analogique-numérique ou l'exécution de l'étape d'enregistrement dans une période dans laquelle le signal analogique ou le signal numérique basé sur une onde ultrasonique en provenance de la zone ineffective peut être reçu sur la base des informations enregistrées dans la seconde mémoire.

[Fig. 1]

FIG. 1

[Fig. 2]

LIGHT
IRRADIATION

↓

t0                    t1

Sig1

Sig2

Sig3

FIG. 2

[Fig. 3]

FIG. 3

[Fig. 4]

LIGHT
IRRADIATION

↓

t0                                    t1  t2 t3  t4

Sig5

Sig1

Sig6

Sig2

Sig3

FIG. 4

[Fig. 5]

ULTRASONIC
WAVE

↓

t0                          t1  t2 t3  t4

Sig5

Sig1

Sig6

Sig2

Sig3

FIG. 5

[Fig. 6]

FIG. 6

[Fig. 7]

FIG. 7

[Fig. 8]

FIG. 8

[Fig. 9]

FIG. 9

[Fig. 10]

```
          ┌─────────────┐
          │    START    │
          └──────┬──────┘
                 │
    ┌────────────▼────────────┐          S101
    │        SET DELAY        │
    └────────────┬────────────┘
                 │
    ┌────────────▼────────────┐          S102
    │       SET PERIOD        │
    └────────────┬────────────┘
                 │
       ┌─────────▼─────────┐
   NO ◄│      LIGHT         │             S103
       │   IRRADIATION ?    │
       └─────────┬─────────┘
               YES
                 │
    ┌────────────▼────────────┐          S104
    │   CONVERT ANALOG SIGNAL │
    │           AND           │
    │      RECORD IN FIFO     │
    └────────────┬────────────┘
                 │
       ┌─────────▼─────────┐
   NO ◄│     200 DATA ?     │             S105
       └─────────┬─────────┘
               YES
                 │
    ┌────────────▼────────────┐          S106
    │ FIFO MEMORY → CPU TRANSFER │
    └────────────┬────────────┘
                 │
          ┌──────▼──────┐
          │     END     │
          └─────────────┘
```

# FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008081558 A1
- US 2005288581 A1